# FASCICULE DE BREVET EUROPEEN

(11) **EP 3 075 302 B1**
(45) Date de publication et mention de la délivrance du brevet: **24.10.2018**
(21) Numéro de dépôt: 16020104.2
(22) Date de dépôt: 01.04.2016
(51) Int. Cl.: A61B 1/267

(54) **ELEMENTS DE POSITIONNEMENT, DE VISEE,DE GUIDAGE ET DE MAINTIEN DE LA SONDE D'INTUBATION POUR LAME DE LARYNGOSCOPE**
ELEMENTE ZUM POSITIONIEREN, ZIELEN, FÜHREN UND HALTEN DER INTUBATIONSSONDE FÜR EINEN LARYNGOSKOPSPATEL
ELEMENTS FOR POSITIONING, AIMING, GUIDING AND HOLDING THE INTUBATION PROBE FOR LARYNGOSCOPE BLADE

(30) Priorité: 02.04.2015 FR 1500690
(43) Date de publication de la demande: 05.10.2016
(73) Titulaire: Jacques, Vincent, 11100 Narbonne (FR)
(72) Inventeur: Jacques, Vincent, 11100 Narbonne (FR)

(56) Documents cités:
- FR-A1- 3 009 944
- US-A1- 2011 196 203
- US-A1- 2013 319 406
- US-B1- 7 128 710

## Description

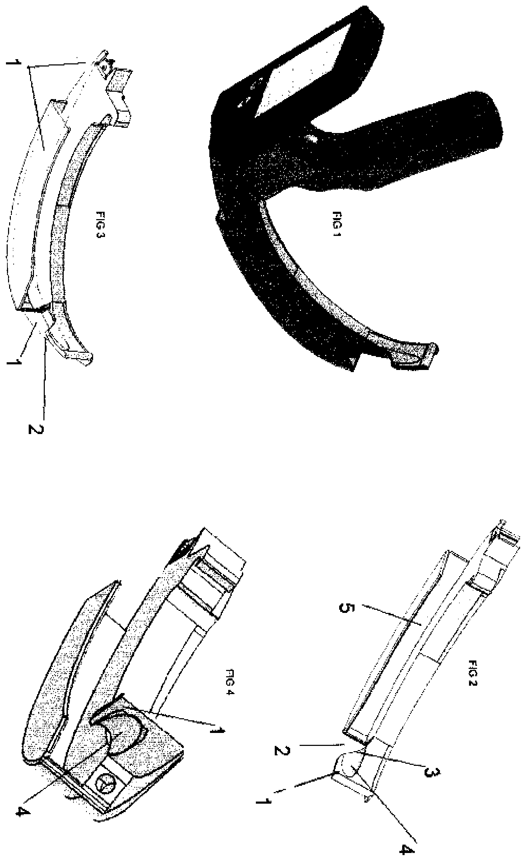

Un laryngoscope ou vidéo-laryngoscope est un instrument utilisé dans le cadre de la procédure d'intubation au cours d'une anesthésie générale et visant à faciliter la mise en place de la sonde d'intubation par laquelle s'effectue la ventilation du patient. Le laryngoscope ou vidéo-laryngoscope comprend en général deux parties assemblées mécaniquement: un manche réutilisable contenant les dispositifs d'éclairage et de traitement de l'image et une lame invasive clipsée sur le manche réutilisable ou à usage unique. La sonde est guidée lors de son introduction par une forme en coin de la partie de la lame libre de contact avec la langue ou bien par un canal ouvert latéralement, dont les parois sont munies de dispositifs qui maintiennent la sonde vers la partie distale de la lame. Des systèmes mécaniques introduits dans la sonde permettent de modifier la courbure de la sonde ou à l'image des endoscopes insérés dans la sonde de guider l'introduction entre les cordes vocales ; Leur usage nécessite le retrait après positionnement de la sonde, montrant par la même l'utilité de l'invention présente qui en supprime avantageusement l'utilisation. Les brevets : EP 2505125 A1 (AIRCRAFT MEDICAL) du 29 avril 2004, US 2002/0087050 (RUDISCHHAUSER) du 4 juillet 2002 et EP 2535076 A1 (DE DOMENICO ANDREA) font état d'un tel dispositif. US 2013/319406 montre une guide d'intubation trachéal avec un canal latéral de guidage. Le brevet 13/01988 (Jacques VINCENT) fait état de lames de laryngoscope dont le canal destiné à guider la sonde trachéale est ouvert du côté de la face de la lame en contact avec la langue. La présente invention est destinée à en améliorer la fonctionnalité. L'utilisation des lames dont le canal ouvert côté langue est rendue difficile, voire impossible dans certains cas, compte tenu de la courbure préformée donnée aux tubes d'intubation ; le maniement du manche du laryngoscope ne permet pas l'alignement de l'extrémité libre de la sonde avec les cordes vocales. La présente invention vise à en assurer le guidage en lui donnant une position contrainte fixe par rapport aux éléments de visée directe ou vidéo. Elle permet d'augmenter la surface en contact avec la base de la langue et par là même entraine une plus grande facilité de maniement du laryngoscope. (fig.1 vidéo laryngoscope équipée d'une lame représentant l'invention).Le canal de guidage de la sonde est plus court que la lame et l'extrémité distale de la lame non adossée au canal (fig.2 - 1) est élargie vers l'axe de la cloison externe du canal latéral, tout en laissant un espace suffisant entre l'extrémité du canal et la plage d'appui sur la langue (fig. 2 -2) afin de permettre le dégagement de la lame une fois la sonde en place. L'extrémité de la sonde pré positionnée (fig.3 -1), vient alors buter sous la plage d'appui, ce qui réduit sa liberté de mouvement et améliore la visée (fig.3 -2).La plage d'appui sur la langue peut être munie d'un coté oblique afin d'augmenter sa surface (fig.1-3) sans obérer le dégagement de la sonde. La plage d'appui sur la langue peut être travaillée en surface conique ouverte vers le canal (fig.2.4- 4), ce qui permet d'améliorer le calage latéral de son extrémité lors de son pré positionnement. La précision de la visée et du guidage sont améliorés, ainsi que le glissement de la sonde.

## Revendications

1. lame de laryngoscope munie d'un canal latéral de guidage d'une sonde, ledit canal est ouvert du côté de la langue, le canal étant plus court que la lame (5) et muni d'une cloison externe, **caractérisée en ce que** l'extrémité distale de la lame, qui n'est pas adossée au canal et qui est destinée à venir en appui sur la langue (1), est élargie vers l'axe de la cloison externe du canal latéral, tout en laissant un espace suffisant au dégagement de la sonde entre l'extrémité du canal et la plage d'appui sur la langue (2).

2. Lame de laryngoscope suivant la revendication 1 dont la plage d'appui à son extrémité distale est munie d'un côté s'évasant en oblique au regard du canal (3).

3. Lame de laryngoscope suivant la revendication 1 dont la plage d'appui, élargie à son extrémité distale, est travaillée en surface conique ouverte vers le canal (4).

## Patentansprüche

1. LARYNGOSKOP-SPATEL, AUSGESTATTET MIT EINEM LATERALEN FÜHRUNGSKANAL FÜR EINE SONDE, DIESER KANAL IST AUF DER SEITE DER ZUNGE GEÖFFNET, DIESER KANAL IST KÜRZER ALS DER SPATEL **(5)** UND MIT EINER ÄUSSEREN TRENNWAND VERSEHEN, DIE DADURCH CHARAKTERISIERT IST, DASS DAS DISTALENDE DES SPATELS, DAS NICHT AUF DEM KANAL AUFLIEGT UND DAS SICH AUF DER ZUNGE ABSTÜTZEN SOLL **(1),** IN RICHTUNG DER ACHSE DER ÄUSSEREN TRENNWAND DES LATERALEN FÜHRUNGSKANALS VERLÄNGERT WIRD, GLEICHZEITIG JEDOCH AUSEICHEND PLATZ FÜR DIE HERAUSNAHME DER SONDE ZWISCHEN DEM ENDE DES KANALS UND DEM AUFLAGEBEREICH AUF DER ZUNGE BIETET.

2. LARYNGOSKOP-SPATEL ENTSPRECHEND PUNKT 1, DESSEN AUFLAGEBEREICH SICH AM DISTALENDE ZUM KANAL HIN SCHRÄG ERWEITERT(3).

3. LARYNGOSKOP-SPATEL ENTSPRECHEND PUNKT 1, DESSEN AUFLAGEBEREICH, DER AM DISTALENDE VERLÄNGERT IST, EINE KONISCHE, ZUM KANAL HIN OFFENE FLÄCHE, BILDET(4).

## Claims

1. LARYNGOSCOPE BLADE COMPRISING A SIDE CHANNEL GUIDING A PROBE, SAID CHANNEL, SHORTER THAN THE BLADE (5) AND DESIGNED WITH AN EXTERNAL WALL, IS OPENED ON THE TONGUE SIDE, **CHARACTERIZED IN THAT** THE DISTAL END OF THE BLADE NOT RESTING AGAINST THE CHANNEL AND INTENDED TO BE A TONGUE CONTACT AREA (1) IS WIDENED TOWARDS THE AXIS OF THE EXTERNAL WALL OF THE SIDE CHANNEL, WHILE LEAVING A SPACE SUFFICIENT FOR THE CLEARANCE OF THE PROBE BETWEEN THE END OF THE CHANNEL AND THE TONGUE CONTACT AREA.

2. LARYNGOSCOPE BLADE ACCORDING TO CLAIM 1, WHEREIN THE TONGUE CONTACT AREA AT ITS DISTAL END IS SHAPED OBLIQUELY FACING THE SIDE CHANNEL (3).

3. LARYNGOSCOPE BLADE ACCORDING TO CLAIM 1, WHEREIN THE TONGUE CONTACT AREA AT ITS DISTAL END IS SHAPED INTO AN OPENED CONICAL SURFACE IN FRONT OF THE SIDE CHANNEL (4).
